# EUROPÄISCHE PATENTSCHRIFT

(11) **EP 1 570 806 B1**
(45) Veröffentlichungstag und Bekanntmachung des Hinweises auf die Patenterteilung: **03.03.2010**
(21) Anmeldenummer: 05011897.5
(22) Anmeldetag: 16.03.2000
(51) Int. Cl.: A61F 2/00

(54) **Flächiges Implantat, Verfahren zu seiner Herstellung und Verwendung in der Chirurgie**
Prosthetic patch, method for its manufacture and its use in surgery
Patch prothétique, sa méthode de fabrication et son utilisation en chirurgie

(30) Priorität: 20.03.1999 DE 19912648
(43) Veröffentlichungstag der Anmeldung: 07.09.2005
(62) Teilanmeldung aus: 00105616.7
(73) Patentinhaber: Aesculap AG, 78532 Tuttlingen (DE)
(72) Erfinder: Goldmann, Helmut, Dr., 78532 Tuttlingen (DE)
(74) Vertreter: Patentanwälte Ruff, Wilhelm, Beier, Dauster & Partner

(56) Entgegenhaltungen:
- WO-A-89/08467
- WO-A-90/14810
- WO-A-92/10218
- WO-A-98/49967
- WO-A-99/51163
- CA-A- 2 114 282
- FR-A- 2 145 975

## Beschreibung

Die vorliegende Erfindung betrifft ein flächiges Implantat, und ein Verfahren zu seiner Herstellung.

Der Eingeweidebruch, die sogenannte Hernie, stellt eine häufige Erkrankung dar. Hierbei handelt es sich im allgemeinen um ein Hindurchtreten von Organen oder Organteilen aus der natürlichen Körperhöhle durch eine vorgebildete oder erworbene Lücke. Unter den äußeren Brüchen, bei denen stets der Bruchsack vom Bauchfell umschlossen wird, sind Leisten-, Nabel- und Narbenbrüche die häufigsten Formen. Ursachen für das Auftreten von Hernien sind vor allem Muskel- oder Bindegewebsschwächen in Zusammenhang mit Überbelastungen, altersbedingter Erschlaffung, angeborener Schwächung der Bauchdecke oder ungenügende Narbenbildung nach einem Leibesschnitt (Narbenbruch).

Eine effektive Behandlung ist in den meisten Fällen durch einen operativen Eingriff möglich, bei dem der Bruchinhalt aus dem Bruchsack in den Bauch zurückverlagert wird und die Bruchpforte verschlossen wird. Dieser Verschluss der Bruchpforte erfolgt konventionell durch eine Naht.

Diese chirurgische Technik hat jedoch den Nachteil, dass es in bis zu 20 % der Fälle zum erneuten Bruch, dem sogenannten Bruchrezidiv kommen kann.

Wegen dieser unbefriedigenden Rezidivrate nach der konventionellen Hernienoperation werden in der modernen Hernienchirurgie zunehmend künstliche Verstärkungsmaterialien zur Bauchwandrekonstruktion verwendet. Hier spielen besonders Polypropylen- aber auch Polyesternetze eine Rolle.

Obwohl die Verwendung solcher Netze offensichtlich zu einer deutlichen Verringerung der Rezidivrate geführt hat, sind diese Implantate nicht unproblematisch wegen möglicher Infektionen oder Fistelbildungen, besonders aber wegen des Risikos von Weichteiladhäsionen.

Bei den bisherigen Implantaten handelt es sich um offene textile Strukturen, die ein Anhaften von Zellen sowie ein Durchwachsen von Zellen begünstigen. Dies ist einerseits von Vorteil, da es eine feste Verbindung des Implantats mit der Bauchdecke ausbildet und somit die gewünschte Stützfunktion gewährleistet. Andererseits kann es zu schwer lösbaren Verwachsungen im Bauchraum mit Verhärtungen (Narbenpanzer), Beeinträchtigungen des Darms sowie der inneren Organe und damit verbundenen Beschwerden beim Patienten führen.

Es sind auch flächige textile Implantate bekannt, die mit einem Imprägnierungsmittel versiegelt sind. Hier zeigen sich aber Probleme bei der Fixierung an der Bauchwand, insbesondere bei langandauerndem Verbleib im Körper.

Ein flächiges implantat zur Verwendung in der Chirurgie gemäß den Merkmalen des Oberbegriffs des Anspruchs 1 ist aus dem Dokument FR 2145975 bekannt.

Es stellt sich die Aufgabe, ein Implantat zum Einsatz bei chirurgischen Eingriffen zur Verfügung zu stellen, das die Schwierigkeiten von Implantaten aus dem Stand der Technik überwindet, das einfach und kostengünstig herzustellen und nach üblichen chirurgischen Methoden einsetzbar ist.

Diese Aufgabe wird gelöst durch ein flächiges Implantat nach Anspruch 1 und seine Herstellung nach den Ansprüchen 12 und 13. Das erfindungsgemäße Implantat erlaubt eine gute Verankerung von Körperzellen auf der der Bauchwand zugewandten strukturierten Seite und verhindert ein unerwünschtes Verwachsen mit Organen und Körperteilen auf der dem Bauchinnenraum zugewandten im wesentlichen geschlossenen Seite.

Zur Verwendung in der Chirurgie stellt die Erfindung ein flächiges Implantat zur Verfügung mit einem flexiblen Flächengebilde, das auf einer Seite eine im wesentlichen geschlossene Oberfläche aus Polyurethan besitzt und auf der anderen Seite eine dreidimensionale Mikrostruktur, die ein Einwachsen von Zellen erlaubt. Insbesondere kann die im wesentlichen geschlossene Oberfläche mikroporös sein, wobei die Mikroporen so klein sind, dass sie einen Stoffaustausch erlauben, aber das Einwachsen von Zellen im wesentlichen verhindern.

Die im wesentlichen geschlossene Oberfläche ist glatt ausgebildet. Ferner ist die im wesentlichen geschlossene Oberfläche von einer mit dem flexiblen Flächengebilde verbundenen Oberflächenschicht, insbesondere einer Beschichtung des Flächengebildes ausgebildet.

Erfindungsgemäß besitzt die dreidimensionale Mikrostruktur hintergreifbare Stellen zum Einwachsen von Zellen. Mit Vorteil kann das flexible Flächengebilde von einem porösen flexiblen Strukturmaterial, insbesondere einem flexiblen Träger gebildet sein und die dreidimensionale Mikrostruktur von der freiliegenden Oberflächenstruktur des Strukturmaterials gebildet sein. Als Beispiele für poröses flexibles Strukturmaterial sind offenzelliger Strukturschaum oder eine Gitterstruktur zu nennen.

Mit Vorteil kann das Implantat aus mindestens einem synthetischen Polymermaterial gebildet sein. Bevorzugt kann das Flächengebilde des Implantats gemäß der Erfindung aus Polypropylen, Polyester, Polytetrafluorethylen und/oder Polyester und Polytetrafluorethylen gebildet sein. Gemäß der Erfindung besonders bevorzugt ist ein flächiges Implantat aus Polyester und Polytetrafluorethylen. Es können auch resorbierbare Materialien, wie Polylactide, Polyglycolide und Copolymere davon Anwendung finden, wenn eine Resorbierbarkeit bzw. Teilresorbierbarkeit erwünscht ist.

In einer Ausführungsform der Erfindung kann das flexible Strukturmaterial ein Textilmaterial, insbesondere ein poröser textiler Träger sein. Bevorzugt kann der flexible textile Träger mindestens auf der Seite mit der dreidimensionalen Mikrostruktur eine bei Gefäßimplantaten bekannte offene Textilstruktur aufweisen, die insbesondere von texturierten Garnen, Flottungen und/oder Veloursschlingen gebildet ist, wie sie beispielsweise aus den Veröffentlichungen US 4047252, US 3878565, DE 2461370 und US 4517687 bekannt sind.

Es können schrumpffähige Fasern und daraus hergestellte Garne wie andere Fasern und Garne auch nach üblichen Verfahrenstechniken zu textilen Gebilden verarbeitet werden. Anschließend werden durch eine Schrumpfbehandlung die schrumpffähigen Fasern geschrumpft, was bei daraus hergestellten Textilgebilden zu einer Verdichtung der Struktur führt. Durch den planmäßigen Einsatz von schrumpffähigen Fasern und nicht schrumpffähigen Fasern in Kombination können gezielt Modifikationen der Textilstruktur vorgenommen werden.

Erfindungsgemäß kann das Flächengebilde nach einer textilen Technik, insbesondere Wirken, Stricken, Weben oder Flechten ausgebildet sein. Solche Verfahrenstechniken sind den Fachleuten bekannt, so dass auf eine detaillierte Ausführung hier verzichtet wird. Auf diese Weise ist eine einfache und kostengünstige Herstellung nach bekannten und in der Praxis bewährten Verfahrenstechniken und mit üblichen Maschinen und Werkzeugen möglich.

Mit Vorteil kann das flexible textile Flächengebilde, insbesondere der textile Träger, eine Webware oder eine insbesondere gewirkte Maschenware sein, die mindestens auf der dreidimensional strukturierten Seite freiliegende, als Verankerung für Zellen dienende Faser oder Fäden aufweist.

Es können textile Fasermaterialien wie synthetische Monofilamente, multifile Fäden oder multifile Garne verwendet werden. Gemäß der Erfindung bevorzugt kann das Flächengebilde mindestens teilweise mit multifilen Garnen ausgebildet sein. Diese Garne können glatt oder texturiert sein. In einer Ausführungsform der Erfindung können die Garne aus nur einer Art von Fasermaterial gebildet sein. In einer anderen Ausführungsform können die Garne aus mehreren Fasermaterialien gebildet sein. Die Garne können mindestens zum Teil aus hoch schrumpffähigem Fasermaterial gebildet sein. Sie können auch Mischungen aus nichtresorbierbaren und resorbierbaren Fasermaterialien sein.

Das erfindungsgemäße Implantat kann sich insbesondere dadurch auszeichnen, dass eine Fläche mit einer strukturierten Oberfläche ausgebildet ist. In einer bevorzugten Ausführungsform kann die strukturierte Oberfläche als Velours ausgebildet sein. In einer besonders bevorzugten Ausführungsform der Erfindung kann das Flächengebilde als Velours, insbesondere als Einfachvelours ausgebildet sein. In Weiterbildung der besonders bevorzugten Ausführungsform kann das Flächengebilde als Doppelvelours ausgebildet sein. Insbesondere kann der Doppelvelours mit unterschiedlichen Polhöhen auf beiden Seiten des Flächengebildes ausgebildet sein.

In einer bevorzugten Ausführungsform der Erfindung kann das flexible Flächengebilde, insbesondere der texturierte Träger ein Velours, insbesondere ein Doppelvelours sein, wobei ein Doppelvelours an der strukturierten Seite vorzugsweise eine größere Polhöhe aufweist als auf der im wesentlichen geschlossenen Seite des Implantats.

Erfindungsgemäß kann sich das flächige Implantat dadurch auszeichnen, dass die der strukturierten Oberfläche gegenüberliegende Seite als glatte Oberfläche ausgebildet ist. Glatt bedeutet in diesem Fall, dass die Oberfläche keine Strukturierung, z. B. durch die Textilkonstruktion aufweist, die eine Verankerung von Zellverbänden ermöglicht. Ferner bedeutet glatt, dass keine Fasern des verwendeten Textilmaterials aus der Fläche herausragen.

In einer anderen Ausführungsform der Erfindung kann das Implantat von einem dreidimensionalen Vlies gebildet sein, wobei Fasermaterialien auf der Seite mit im wesentlichen geschlossener Oberfläche dicht beieinander liegen und auf der Seite mit Mikrostruktur einen offenen Verbund bilden.

Die im wesentlichen geschlossene Oberfläche kann vollständig dicht sein. Ist sie, was bevorzugt ist, mikroporös, dann zeichnet sie sich im Vergleich zu der Porosität des Flächengebildes durch eine um mindestens eine 10er Potenz geringere Porosität aus. Insbesondere die erfindungsgemäß bevorzugte Sprühschicht weist eine Tiefenporosität auf.

Mit Vorteil kann die im wesentlichen geschlossene Oberfläche und insbesondere das gesamte Implantat eine Luftdurchlässigkeit von 5 bis 100 ml Luft/cm^{2.}min, insbesondere 25 bis 75 ml Luft/cm^{2.}min besitzen, bei einer Druckdifferenz von 1,2 kPascal. Bei einer solchen Mikroporosität ist ein Stoffaustausch im molekularen Bereich möglich. Dies begünstigt Stoffwechselvorgänge im Bereich des Implantats, fördert die Zufuhr von lebenswichtigen Nährstoffen und Aufbaustoffen sowie die Abfuhr von Stoffwechselschlacken und Schadstoffen. Auf diese Weise ist eine gute Verträglichkeit und erfolgreiches Heilen vorteilhaft möglich. Die Mikroporosität der im wesentlichen geschlossenen Oberfläche ist jedoch nicht geeignet für den Durchtritt bzw. die feste und durchgehende Verankerung von großen Partikeln wie Zellen.

Die erfindungsgemäße im wesentlichen geschlossene Oberfläche führt zu einer Verminderung der Strukturierung der Implantatoberfläche auf einer Seite. Erhebungen und Vertiefungen, die sich beispielsweise aus der Herstellungstechnik des textilen Flächengebildes ergeben, werden durch die im wesentlichen geschlossene, insbesondere glatte Oberfläche ausgeglichen. Außerdem werden einzelne Fasern, die aus dem textilen Flächengebilde hervorstehen eingeschlossen. Das Schließen der Implantatoberfläche gemäß der Erfindung kann dementsprechend als eine Art Oberflächenversiegelung betrachtet werden.

Auf diese Weise ist die im wesentlichen geschlossene Oberfläche mit ihrer geringen Porosität und geringen Oberflächenstruktur ungünstig für ein Anhaften von Zellen. Die Zellen finden keine geeigneten Verankerungsstellen, die für ihr Wachstum erforderlich sind. Auf diese Weise ist eine Besiedelung der im wesentlichen geschlossenen Implantatoberfläche mit Zellen im wesentlichen zu verhindern.

Ferner kann das erfindungsgemäße Implantat bevorzugt ausfranssichere Ränder aufweisen, die ausfranssicher verarbeitet und/oder gebunden, insbesondere verschweißt sind.

Mit Vorteil kann das Implantat eine Gesamtdicke von ca. 0,1 bis 1,2 mm besitzen, wobei die Dicke der im wesentlichen geschlossenen Oberfläche 3 bis 15 % davon beträgt. Außerdem kann der textile Träger eine gewebte, gewirkte oder gestrickte Grundbindung mit einer Dicke von 0,05 bis 0,4 mm besitzen und eine mindestens einseitige offene Struktur mit einer Dicke von 0,05 bis 0,8 mm.

Es ist erfindungsgemäß vorgesehen, dass alle Komponenten des Implantats bioverträglich und langzeitstabil sind. Auf diese Weise kommt es nach der Implantation im physiologischen Milieu des Körpers nicht zu einem Abbau der Oberfläche. Die im wesentlichen geschlossene Oberfläche des Implantats behält somit ihre zellabweisende Eigenschaft, die ein Anhaften und Einwachsen von Zellen verhindert, wie es gemäß der Erfindung vorgesehen ist.

Das Implantat ist gemäß der Erfindung aus flexiblem Material. Erfindungsgemäß ist die im wesentlichen geschlossene Oberfläche in Form einer Lösung in einem niedrigsiedenden Lösemittel auf ein insbesondere textiles Flächengebilde einseitig aufgetragen und die im wesentlichen geschlossene Oberfläche des flächigen Implantats ist aus unvernetztem Polyurethan gebildet.

In Weiterbildung kann im Implantat ein antimikrobiotischer Wirkstoff, wie beispielsweise ein Antibiotikum enthalten sein. Die Verabreichung von Antibiotika dient insbesondere der Vorbeugung von Infektionen. Zur Prophylaxe und Therapie mit Antibiotika finden im Bereich der Chirurgie beispielsweise Cefalosporine wie Cefazolin oder Cefamandol, Netilmicin, Penicilline wie Oxacillin oder Mezlocillin, Tetracyclin, Metronidazol oder Aminoglykoside wie Gentamicin oder Neomycin sowie z. B. Rifampicin Anwendung. Die Fachleute können gemäß den jeweiligen Erfordernissen einen oder mehrere geeignete Wirkstoffe zur Anwendung auswählen. Auch Wachstumsfaktoren können im Implantat enthalten sein.

Die vorliegende Erfindung betrifft ferner ein Verfahren zur Herstellung eines Implantats zur Verwendung in der Chirurgie, das umfasst Ausbilden einer einseitigen im wesentlichen geschlossenen Oberfläche aus Polyurethan auf einer Seite eines porösen, textilen Flächengebildes zur Verhinderung des Einwachsens von Zellen.

In einer anderen Ausführungsform betrifft die Erfindung ein Verfahren zur Herstellung eines Implantats zur Verwendung in der Chirurgie durch Bilden einer im wesentlichen geschlossenen Oberflächenschicht aus Polyurethan und einseitiges, insbesondere aufbauendes Ausbilden einer mit dieser verbundenen dreidimensionalen Mikrostruktur.

In das erfindungsgemäße Verfahren wird die im wesentlichen geschlossene Oberfläche durch Beschichten, insbesondere Sprühbeschichten ausgebildet. In das erfindungsgemäße Verfahren wird Polyurethan aus einer Lösung in niedrigsiedendem organischem Lösemittel aufgesprüht. Als Beispiele für geeignete Lösemittel sind Chloroform oder Methylenchlorid zu nennen. Durch Verdampfen des Lösemittels bildet sich der Beschichtungsfilm aus. Während der Lösemittelverdampfung können feine Poren in der Oberflächenbeschichtung entstehen, was beim Implantat gemäß der Erfindung von Vorteil ist. Die so ausgebildete Mikroporosität erlaubt den Stoffaustausch im physiologischen Milieu. Die Poren sind jedoch so klein, daß Zellen zurückgehalten werden.

Gemäß der Erfindung ist ein Schließen der Oberfläche zur Verhinderung des Einwachsens von Zellen nur auf einer Seite des flächigen Implantats und nur auf der Oberfläche des Flächengebildes vorgesehen. Mit Vorteil ist die dreidimensional strukturierte Oberfläche des textilen Flächengebildes offen und für ein Einwachsen von Zellen geeignet. Für ein Anhaften und Anwachsen von Zellen ist die poröse bzw. strukturierte Oberfläche geeignet, die ein hintergreifendes Einwachsen der Zellen ermöglicht.

Zur Verwendung in der Chirurgie kann das erfindungsgemäß modifizierte Implantat in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfasst die Behandlung mit ionisierender Strahlung wie beispielsweise Bestrahlung mit β-Strahlen oder γ -Strahlen, im Bereich von 0,1 Mrad bis 10 Mrad, insbesondere 0,8 Mrad bis 2,5 Mrad.

Des weiteren betrifft die Erfindung auch die Verwendung eines Implantats in der Chirurgie, insbesondere zur Behandlung von Wanddefekten in Körperhöhlen, besonders von Bauchwanddefekten.

Zu diesem Zweck kann das erfindungsgemäße modifizierte Implantatmaterial auf eine gewünschte Größe und Form zurechtgeschnitten werden. Mit Vorteil kann das chirurgische Implantat gemäß der Erfindung in zweckmäßiger Dimension zugeschnitten gebrauchsfertig in geeigneter Weise verpackt vorliegen.

Das Einbringen des erfindungsgemäßen flächigen Implantats in den Bauchraum erfolgt in der Weise, dass die Seite des Implantats, deren Oberfläche zur Verhinderung des Einwachsens von Zellen modifiziert ist, zur Bauchinnenseite hin, d. h. den Eingeweiden zugewandt, eingesetzt wird. Die gegenüberliegende Seite des flächigen Implantats, bei der ein Einwachsen von Zellen möglich ist, wird der Bauchdeckenseite zugewandt eingesetzt.

Auf diese Weise können während des Heilungsprozesses von der Bauchdecke her Körperzellen auf der Oberfläche des Implantats anhaften, in die Oberflächenstruktur eindringen und im Laufe der Zeit zu einem Verwachsen des Implantats mit der Bauchdecke führen. Dies führt zu einem sicheren Verbund von Bauchdecke und Implantat, was zur Stabilisierung der Bauchwand beiträgt und damit den Behandlungserfolg sichert.

Die im wesentlichen geschlossene Oberfläche zur Verhinderung des Einwachsens von Zellen auf der Bauchinnenseite des Implantats ist für von der Bauchdeckenseite einwachsende Zellen nicht durchdringbar.

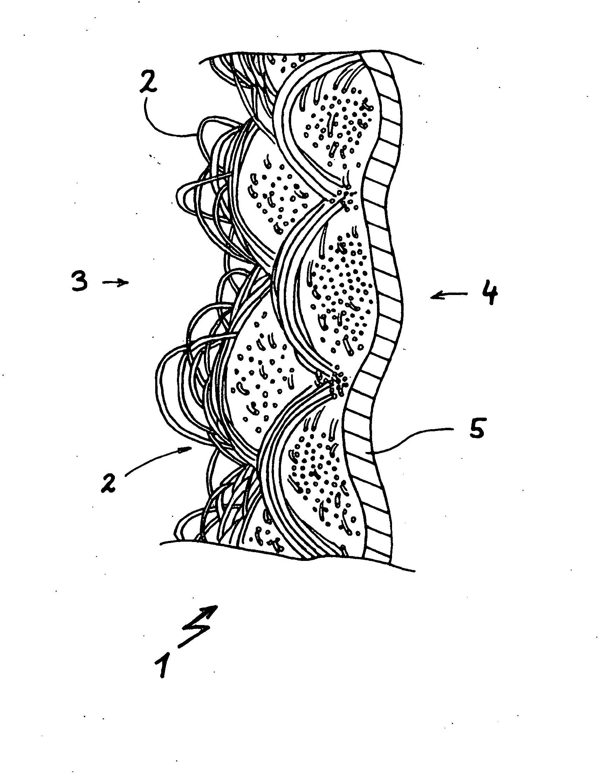

Weitere Merkmale der Erfindung ergeben sich aus der nachfolgenden Beschreibung eines bevorzugten Beispiels in Verbindung mit den Unteransprüchen und der Zeichnung. Hierbei können die einzelnen Merkmale jeweils für sich alleine oder zu mehreren bei einer Ausführungsform der Erfindung verwirklicht sein. Das Beispiel dient jedoch nur zur Erläuterung und ist nicht als Einschränkung zu verstehen.

Die Zeichnung zeigt eine schematischen Schnitt durch ein erfindungsgemäßes Implantat.

### Beispiel 1

Ein textiles Flächengebilde 1 ist als Kettengewirk aus multifilem Polyethylenterephthalatgarn in Form eines Einfachvelours ausgebildet, wobei Veloursschlingen 2 aus texturiertem Garn bestehen. Das Gewirk kann als Doppelveloursgewirk ähnlich ausgebildet sein, wie die Gewirke nach den US-Patentschriften 4047252 und 4193397. Es ist jedoch im Gegensatz zu den dort beschriebenen rohrförmigen Kettenwirkstoffen flächig ausgebildet. Es kann auch als Einfachvelours ausgebildet sein. Das Gewirk ist porös und flexibel. Es besitzt auf der Veloursseite 3 eine offene dreidimensional strukturierte Oberfläche, die aufgrund der Veloursschlingen und der Texturierung der Fasern, zahlreiche im wesentlichen gleichmäßig über die Oberfläche verteilte Hintergreifmöglichkeiten zum Einwachsen von Körperzellen aufweist. Dabei sind die Öffnungen zwischen den Garnschlingen bzw. den einzelnen Fasern groß im Verhältnis zur Größe von Körperzellen. Dies ermöglicht das Einwachsen eines zusammenhängenden Zellverbundes.

Die gegenüberliegende Seite 4 des Gewirks 1 ist aufgrund der Bindung des Gewirks dichter und eher eben. Zusätzlich weist das Gewirk 1 an dieser Seite eine Sprühbeschichtung 5 aus unvernetztem Polyurethan auf, die mit den an der Oberfläche freiliegenden Fasern des Gewirks 1 verbunden ist und die textile Struktur an dieser Oberfläche verschließt. Die Dicke der Sprühbeschichtung liegt in der Größenordnung von etwa 1/10 bis 1/20 der Gesamtdicke von textilem Flächengebilde und Versiegelungsschicht, das heißt, der Gesamtdicke des flächigen Implantats von ca. 0,8 mm.

Durch die Technik der Sprühbeschichtung, das heißt durch Aufsprühen einer Lösung des Polyurethans in Chloroform hat die Schicht 5 einen Aufbau, der mit einem dichten Vlies in etwa vergleichbar ist, mikroporös ist und aufgrund der elastomeren Eigenschaften des Polyurethans flexibel ist. Als Polyurethan eignen sich beispielsweise bekannte lineare Polyester und Polyurethane. Durch die Versiegelungsschicht 5 ist das textile Flächengebilde 1 aus dem Veloursgewirk an der von den Veloursschlingen 2 abweisenden Seite nach Art eines Tiefenfilters im wesentlichen verschlossen. Die Mikroporen, deren Größe in etwa in der Gröβenordnung der Größe von Körperzellen oder kleiner ist, erlauben einen Stoffaustausch von Körperflüssigkeiten zur Aufrechterhaltung des Stoffwechsels, erlauben jedoch nicht das Einwachsen von Zellen, zumindest nicht in solcher Form, dass ein Anwachsen an Körperteile im Bereich der Bauchhöhle zu befürchten ist.

Die Versiegelungsschicht 5 dringt nur unwesentlich in die textile Oberfläche des textilen Trägers 1 ein, so dass das dreidimensionale Volumen des Flächengebildes zum Einwachsen von Zellen aus der Bauchwand zur Verfügung steht, während die im wesentlichen geschlossene Versiegelungsschicht 5 ein solches Einwachsen von Zellen auf der Seite des Bauchraumes verhindert.

Die Ränder des Flächengebildes sind aufgrund der Wirkart an sich schon ausfransfest. Die Versiegelungsschicht verhindert ein Einrollen des Gewirks und vermindert zusätzlich die Neigung zur Fransenbildung. Falls gewünscht, können die Ränder zusätzlich noch durch Verschweißen der thermoplastischen Fasern ausfransfest gemacht werden. Zur Verwendung in der Chirurgie werden Stücke von etwa 8 x 2 cm bis 30 x 30 hergestellt. Je nach Bedarf können diese Stücke vor dem Implantieren noch zurechtgeschnitten werden.

Mit dem erfindungsgemäßen Implantat durchgeführte Tierversuche ergaben gute Ergebnisse. Auf der unbeschichteten, offenen Seite der Implantate erfolgte ein sehr guter Einbau in das Gewebe mit kräftiger Vaskularisation in den Implantatmaschen und völlig fehlenden Abstoßungserscheinungen. Durch die Versiegelung der bauchhöhlenseitigen Fläche zeigten sich an dieser Seite keine Verwachsungen. Auch nach längerer Verweildauer des Implantats im Bauchraum war eine freie Beweglichkeit der Bauchorgane relativ zum Implantat gewährleistet.

## Patentansprüche

1. Flächiges Implantat (1) zur Verwendung in der Chirurgie mit einem flexiblen textilen Flächengebilde, das auf einer Seite eine zur Verhinderung des Einwachsens von Zellen im wesentlichen geschlossene glatte Oberfläche (5) aus einem Polymer besitzt und auf der anderen Seite eine dreidimensionale Mikrostruktur (3), die ein Einwachsen von Zellen erlaubt und hintergreifbare Stellen zum Einwachsen von Zellen besitzt, **dadurch gekennzeichnet, dass** die im wesentlichen geschlossene Oberflächenschicht (5) aus Polyurethan durch einseitiges Auftragen einer Lösung von unvernetztem Polyurethan in einem niedrig siedenden Lösungsmittel auf das textile Flächengebilde als Beschichtung ausgebildet ist.

2. Implantat nach Anspruch 1, **dadurch gekennzeichnet, dass** die im wesentlichen geschlossene Oberfläche (5) mikroporös ist, wobei die Mikroporen so klein sind, dass sie einen Stoffaustausch erlauben, aber das Einwachsen von Zellen im wesentlichen verhindern.

3. Implantat nach einem der vorhergehenden Ansprüche, **dadurch gekennzeichnet, dass** die im wesentlichen geschlossene Oberfläche (5) von einer mit dem flexiblen Flächengebilde verbundenen Oberflächenschicht, insbesondere einer Beschichtung des Flächengebildes, gebildet ist.

4. Implantat nach einem der vorhergehenden Ansprüche, **dadurch gekennzeichnet, dass** das flexible textile Flächengebilde von einem porösen flexiblen Strukturmaterial, insbesondere einem flexiblen Träger, gebildet ist und die dreidimensionale Mikrostruktur (3) von der

5. Implantat nach Anspruch 4, **dadurch gekennzeichnet, dass** das flexible Strukturmaterial ein Textilmaterial, insbesondere ein poröser textiler Träger ist, wobei das flexible Textilmaterial, insbesondere der textile Träger mindestens auf der Seite mit der dreidimensionalen Mikrostruktur (3) eine bei Gefäßimplantaten bekannte offene Textilstruktur aufweist, die insbesondere von texturierten Garnen, Flottungen und/oder Velourschlingen gebildet ist. freiliegenden Oberflächenstruktur des Strukturmaterials gebildet ist.

6. Implantat nach einem der vorhergehenden Ansprüche, **dadurch gekennzeichnet, dass** das flexbile Flächengebilde, insbesondere der textile Träger, eine Webware oder eine insbesondere gewirkte Maschenware ist, die mindestens auf der dreidimensional strukturierten Seite freiliegende, als Verankerung für Zellen dienende Fasern oder Fäden aufweist.

7. Implantat nach einem der vorhergehenden Ansprüche, **dadurch gekennzeichnet, dass** das flexible textile Flächengebilde, insbesondere der texturierte Träger ein Velours, insbesondere ein Doppelvelours ist, wobei ein Doppelvelours an der strukturierten Seite vorzugsweise eine größere Polhöhe aufweist als auf der im wesentlichen geschlossenen Seite des Implantats.

8. Implantat nach einem der vorhergehenden Ansprüche, **dadurch gekennzeichnet, dass** die im wesentlichen geschlossene Oberfläche und insbesondere das gesamte Implantat eine Luftdurchlässigkeit von 5 bis 100 ml Luft/cm^{2.}min, insbesondere 25 bis 75 ml Luft/cm^{2.}min besitzt, bei einer Druckdifferenz von 1,2 kPascal.

9. Implantat nach einem der vorhergehenden Ansprüche, **dadurch gekennzeichnet, dass** es ausfranssichere Ränder aufweist, die ausfranssicher verarbeitet und/oder gebunden, insbesondere verschweißt sind.

10. Implantat nach einem der vorhergehenden Ansprüche, **dadurch gekennzeichnet, dass** es eine Gesamtdicke von ca. 0,1 bis 1,2 mm besitzt, wobei die Dicke der im wesentlichen geschlossenen Oberfläche 3 bis 15 % davon beträgt.

11. Implantat nach einem der vorhergehenden Ansprüche, **dadurch gekennzeichnet, dass** der textile Träger eine gewebte, gewirkte oder gestrickte Grundbindung mit einer Dicke von 0,05 bis 0,4 mm besitzt und eine mindestens einseitige offene Struktur mit einer Dicke von 0,05 bis 0,8 mm, besitzt.

12. Verfahren zur Herstellung des Implantats nach einem der vorhergehenden Ansprüche, **dadurch gekennzeichnet, dass** auf einer Seite eines porösen textilen Flächengebildes (3) zur Verhinderung des Einwachsens von Zellen durch Auftragen einer Lösung von unvernetztem Polyurethan in einem niedrig siedenden Lösungsmittel eine einseitige im wesentlichen geschlossene Oberflächenschicht (5) aus Polyurethan ausgebildet wird.

13. Verfahren nach Anspruch 12, **dadurch gekennzeichnet, dass** die im wesentlichen geschlossene Oberfläche durch Schichtbildung, bevorzugt Beschichten, insbesondere Sprühbeschichten ausgebildet wird.

## Claims

1. Flat implant (1) for use in surgery, with a flexible textile fabric which, on one side, has a substantially closed smooth surface (5), consisting of a polymer, for preventing incorporation of cells, and, on the other side, has a three-dimensional microstructure (3) permitting incorporation of cells and having locations that can be engaged behind to permit incorporation of cells, **characterized in that** the substantially closed surface layer (5) is formed from polyurethane by one-sided application of a solution of uncrosslinked polyurethane in a low boiling solvent to the textile fabric as a coating.

2. Implant according to Claim 1, **characterized in that** the substantially closed surface (5) is microporous, the micropores being sufficiently small that they permit substance exchange but substantially prevent incorporation of cells.

3. Implant according to either of the preceding claims, **characterized in that** the substantially closed surface (5) is formed by a surface layer connected to the flexible fabric, in particular a coating of the fabric.

4. Implant according to one of the preceding claims, **characterized in that** the flexible textile fabric is formed by a porous flexible structure material, in particular a flexible support, and the three-dimensional microstructure (3) is formed by the exposed surface structure of the structure material.

5. Implant according to Claim 4, **characterized in that** the flexible structure material is a textile material, in particular a porous textile support, wherein the flexible textile material, in particular the textile support, at least on the side with the three-dimensional microstructure (3), has an open textile structure which is known in vascular implants and which is formed in particular by textured yarns, float stitches and/or velour loops.

6. Implant according to one of the preceding claims, **characterized in that** the flexible fabric, in particular the textile support, is a woven fabric or an in particular knitted mesh fabric which, at least on the three-dimensionally structured side, has exposed fibres or filaments used as anchoring for cells.

7. Implant according to one of the preceding claims, **characterized in that** the flexible textile fabric, in particular the textured support, is a velour, in particular a double velour, and a double velour on the structured side preferably has a greater pile height than on the substantially closed side of the implant.

8. Implant according to one of the preceding claims, **characterized in that** the substantially closed surface and in particular the entire implant has an air permeability of 5 to 100 ml air/cm² min, in particular 25 to 75 ml air/cm² min, at a pressure difference of 1.2 kPascal.

9. Implant according to one of the preceding claims, **characterized in that** it has fray-proof edges which are processed and/or bonded, in particular welded, in a fray-proof manner.

10. Implant according to one of the preceding claims, **characterized in that** it has a total thickness of ca. 0.1 to 1.2 mm, the thickness of the substantially closed surface amounting to 3 to 15% thereof.

11. Implant according to one of the preceding claims, **characterized in that** the textile support has a woven or knitted basic weave with a thickness of 0.05 to 0.4 mm and has an at least unilaterally open structure with a thickness of 0.05 to 0.8 mm.

12. Method for the manufacture of the implant according to one of the preceding claims, **characterized in that**, in order to prevent the incorporation of cells, a surface layer (5) of polyurethane, substantially closed on one side, is formed on one side of a porous textile fabric (3), by application of a solution of uncrosslinked polyurethane in a low boiling solvent.

13. Method according to Claim 12, **characterized in that** the substantially closed surface is formed by layering, preferably coating, in particular spray coating.

## Revendications

1. Implant plat (1) destiné à être utilisé en chirurgie, doté d'un produit textile plat souple qui possède sur un côté une surface lisse essentiellement fermée (5), constituée d'un polymère et destinée à empêcher la croissance de cellules, et sur l'autre face d'une microstructure tridimensionnelle (3) qui permet la croissance de cellules et possède des emplacements derrière lesquels les cellules peuvent croître,
**caractérisé en ce que**
la couche (5) de surface essentiellement fermée est constituée de polyuréthane et est réalisée en appliquant une solution de polyuréthane non réticulé dans un solvant à bas point d'ébullition placée comme revêtement sur un côté du produit textile plat.

2. Implant selon la revendication 1, **caractérisé en ce que** la surface (5) essentiellement fermée est microporeuse, les micropores étant suffisamment petits pour permettre un échange de matières tout en empêchant essentiellement la croissance de cellules.

3. Implant selon l'une des revendications précédentes, **caractérisé en ce que** la surface (5) essentiellement fermée est constituée d'une couche de surface reliée au produit plat souple et en particulier d'un revêtement du produit plat.

4. Implant selon l'une des revendications précédentes, **caractérisé en ce que** le produit textile plat souple est formé d'un matériau structuré souple poreux, en particulier d'un support souple, et **en ce que** la microstructure tridimensionnelle (3) est formée par la structure de surface libre du matériau structuré.

5. Implant selon la revendication 4, **caractérisé en ce que** le matériau structuré souple est formé d'un matériau textile, en particulier un support textile poreux, le matériau textile souple, en particulier le support textile, présentant au moins sur le côté doté de la microstructure tridimensionnelle (3) une structure textile ouverte connue pour les implants de vaisseaux et formée en particulier de fils texturés, de fils flottés et/ou de boucles velours.

6. Implant selon l'une des revendications précédentes, **caractérisé en ce que** le produit plat souple, en particulier le support textile, est un produit tissé ou un produit en particulier tricoté qui présente sur le côté structuré en trois dimensions des fibres ou des fils libres qui servent d'ancrage pour les cellules.

7. Implant selon l'une des revendications précédentes, **caractérisé en ce que** le produit textile plat souple, en particulier le support texturé, est un velours et en particulier un velours double, le velours double présentant sur le côté structuré des poils de préférence d'une hauteur plus grande que sur le côté essentiellement fermé de l'implant.

8. Implant selon l'une des revendications précédentes, **caractérisé en ce que** la surface essentiellement fermée et en particulier la totalité de l'implant présentent une perméabilité à l'air de 5 à 100 ml/cm².min et en particulier de 25 à 75 ml/cm².min à une différence de pression de 1,2 kPascal.

9. Implant selon l'une des revendications précédentes, **caractérisé en ce qu'**il présente des bords non effilochés, traités de manière à éliminer les franges et/ou collés et en particulier soudés.

10. Implant selon l'une des revendications précédentes, **caractérisé en ce que** son épaisseur totale est d'environ 0,1 à 1,2 mm, l'épaisseur de la surface essentiellement fermée en représentant de 3 à 15 %.

11. Implant selon l'une des revendications précédentes, **caractérisé en ce que** le support textile présente une armure de base tissée, maillée ou tricotée d'une épaisseur de 0,05 à 0,4 mm et sur au moins un côté une structure ouverte d'une épaisseur de 0,5 à 0,8 mm.

12. Procédé de fabrication de l'implant selon l'une des revendications précédentes, **caractérisé en ce que** pour empêcher la croissance de cellules, une couche de surface (5) en polyuréthane, essentiellement fermée, est formée sur un côté d'un produit textile plat poreux (3) par application d'une solution de polyuréthane non réticulé dans un solvant à bas point d'ébullition.

13. Procédé selon la revendication 12, **caractérisé en ce que** la surface essentiellement fermée est obtenue par stratification et de préférence enduction, en particulier enduction par pulvérisation.
